(19) Europäisches Patentamt
European Patent Office
Office européen des brevets

(11) Veröffentlichungsnummer: **0 217 279**
**B1**

(12) **EUROPÄISCHE PATENTSCHRIFT**

(45) Veröffentlichungstag der Patentschrift: **23.01.91**

(51) Int. Cl.$^5$: **C 07 D 209/92**

(21) Anmeldenummer: **86113119.1**

(22) Anmeldetag: **24.09.86**

# (54) Verfahren zur Herstellung von Naphtostyril.

(30) Priorität: **04.10.85 DE 3535482**

(43) Veröffentlichungstag der Anmeldung:
**08.04.87 Patentblatt 87/15**

(45) Bekanntmachung des Hinweises auf die Patenterteilung:
**23.01.91 Patentblatt 91/04**

(84) Benannte Vertragsstaaten:
**CH DE FR GB IT LI**

(56) Entgegenhaltungen:
**PATENT ABSTRACTS OF JAPAN, unexamined applications, C Field, Band 4, Nr. 69, 22. Mai 1980 THE PATENT OFFICE JAPANESE GOVERNMENT Seite 82 C 11**

(73) Patentinhaber: **HOECHST AKTIENGESELLSCHAFT**
**Postfach 80 03 20**
**D-6230 Frankfurt am Main 80 (DE)**

(72) Erfinder: **Arndt, Otto, Dr.**
**Frankfurter Strasse 38**
**D-6238 Hofheim am Taunus (DE)**
Erfinder: **Papenfuhs, Theodor, Dr.**
**Heinrich-Bleicher-Strasse 40**
**D-6000 Frankfurt am Main 50 (DE)**

**EP 0 217 279 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Gegenstand der Erfindung ist ein gegenüber dem Stand der Technik verbessertes Verfahren zur Herstellung von Naphthostyril.

Aus der japanischen bekanntgemachten Patentanmeldung Sho-58-45 425 (JA—OS Sho-55-35 051) ist ein Verfahren zur Herstellung von Naphthostyril der Formel

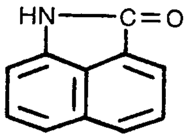

bekannt, wobei eine mittels Natriumhydroxid, Kaliumhydroxid oder vorzugsweise einer Mischung daraus oberhalb 40°C hergestellte wäßrig-alkalische Lösung von 1,8-Naphthalimid auf 15°C bis zur mindestens teilweisen Kristallisation des Naphthalimids abgekühlt und dann bei 20°C mit Hypochlorit in Form von Chlorbleichlauge umgesetzt wird. Das Mengenverhältnis Na:K beträgt hierbei maximal 0,9 und minimal 0,25. Die optimale Reaktionszeit ist mit 2½ Stunden angegeben. Die Ausbeute soll 83% betragen. Der Nachteil dieses Verfahrens liegt in der Anwendung einer sehr hohen Menge Hypochlorit (3—5 Mol pro Mol 1,8-Naphthalimid), die zu Verharzungen und Bildung von Naphthalsäureanhydrid führt.

Aus Chimija i Chimitscheskaja Technologija *4* (1961), 2, 232—237 ist bekannt, daß zu hohe Überschüsse an Hyprochlorit zu Verharzungen führen, welche die Qualität des Produkts, beispielsweise durch Stippen, herabsetzen, was sich jedoch, wie eigene Unterschungen ergeben haben, am Schmelzpunkt nicht bemerkbar macht.

Die Nacharbeitung des in der genannten japanischen Patentanmeldung aufgeführten Ausführungsbeispiels ergab nicht die dort angegebene Ausbeute von 83%, sondern von nur 58%, wobei das erhaltene Produkt einen Reingehalt von 76% (gemäß HPLC = High Performance Liquid Chromatography) aufwies und von dunklen Stippen durchsetzt war. Der geringe Gehalt des Produkts an Naphthalimid (1%) weist auf einen nahezu vollständigen Umsatz hin.

Die Nacharbeitung des Ausführungsbeispiels der genannten japanischen Patentanmeldung ist insofern von Bedeutung, als in diesem Beispiel keinerlei Qualitätsangaben über das erhaltene Naphthostyril, wie Schmelzpunkt, Reingehalt, Nebenkomponenten gemacht werden.

Gemäß der in der zitierten russichen Literaturstelle beschriebenen Verfahrensweise werden die die Ausbeute des Napthostyrils bestimmenden Parameter Konzentration, Temperatur, Reaktionszeit, Überschuß an Alkali (NaOH, KOH) und Überschuß an Hypochlorit (NaOCl + NaCl) optimiert. Die dort beschreibene Verfahrensweise unterscheidet sich jedoch von der Verfahrensweise der genannten japanischen Anmeldung dadurch, daß die Reaktion in hoher Verdünnung (12 l Wasser pro Mol Napthalimid) und bei großem Überschuß an Alkalimetallhydroxid (NaOH + KOH = 12 Mol pro Mol Naphthalimid) in Lösung ablaufen soll, was die Möglichkeit einer geringeren Anwendung von Hypochlorit (1,9 Mol pro Mol Napthalimid) eröffnet. Die optimale Reaktionszeit wird mit 2½ Stunden angegeben. Die Ausbeute soll 85—90% betragen. Der Schmelzpunkt wird mit 174—178°C angegeben. Als weitere Qualitätsangabe findet sich nur der Stickstoffgehalt (7,83%—8,06%) (theoretisch 8,28%). Die stark Verdünnung bedeutet jedoch eine geringe Raumzeitausbeute und der größe Überschuß an Alkali führt zu einer starken Abwasserbelastung durch anorganische Salze, weshalb das inredestehende bekannte Verfahren unwirtschaftlich und umweltbelastend ist.

Es wurde nun gefunden, daß man Naphthostyril unter Vermeidung der den geannten Verfahren anhaftenden Mängel vorteilhaft herstellen kann, indem man das aus der zitierten japanischen Patentanmeldung bekannte Verfahren dahingehend abändert, daß man das 1,8-Napthalimid statt in einer wäßrigen Lösung von Natriumhydroxid und/oder Kaliumhydroxid in einer wäßrigen Lösung von Lithiumhydroxid und Kaliumhydroxid löst und die erhaltene Lösung des Naphthalimids mit maximal 1 Molprozent kristallinem 1,8-Naphthalimid-Natrium, bezogen auf 1 Mol gelöstes Naphthalimid, bei 14°C impft.

Gegenstand der Erfindung ist somit ein Verfahren zu Herstellung von Naphthostyril, welches dadurch gekennzeichnet ist, daß man 1,8-Naphthalimid in einer wäßrigen Lösung von Lithiumhydroxid und Kaliumhydroxid unter Erwärmen auf 40°C bis 100°C, die erhaltene Lösung auf 14°C abkühlt und bei dieser Temperatur mit maximal 1 Molprozent 1,8-Naphthalimid-Natrium, bezogen auf 1 Mol eingesetztes 1,8-Naphthalimid, impft, anschließend Chlorbleichlauge bei 10°C bis 20°C zugibt, wobei sich das Gewichtsmengenverhältnis von eingesetztem 1,8-Napthalimid zu insgesamt vorhandenem Wasser zwischen etwa 1:25 und etwa 1:32 bewegen soll, dann 2 bis 3 Stunden bei 16 bis 20°C nachrührt, nach erfolgter Umsetzung überschüssiges aktives Chlor reduktiv beseitigt, die angefallene wäßrig-alkalische Lösung des Alkalimetallsalzes der gebildeten 1-Amino-naphthalin-8-carbonsäure nach Hochheizen auf 90°C mit etwa 5,9 Mol etwa 30 %iger Salzsäure, bezogen auf 1 Mol eingesetztes 1,8-Naphthalimid, auf den pH-Wert 2,5 bis 2,0 stellt und das hierbei ausfallende Naphthostyril isoliert. Unter der angewandten Chlorbleichlauge ist ein äquivalentes Gemisch aus Natriumhypochlorit und Natriumchlorid zu verstehen, wie in Gmelins Handbuch der Anorganischen Chemie 6, 8. Auflage (1972), Seite 293 bzw. Ullmanns Enzyklopädie der Technischen Chemie *9*, 4. Auflage (1975), Seite 544 beschreiben.

Die Vorteile des erfindungsgemäßen Verfahrens gegenüber den genannten bekannten Verfahren liegen darin, daß (1) das Naphthostyril in besserer Qualität, d.h. in einem höheren Reinheitsgrad von 92—95% und zudem stippenfrei anfällt, (2) wegen der anzuwendenden geringen Menge an Hypochlorit weniger Sulfit zur Zerstörung überschüssigen aktiven Chlors erforderlich ist, wodurch eine Sulfat- und Chlorid-Entlastung des Abwassers gegeben ist, und (3) trotz der Anwendung von weniger Hypochlorit die optimale Reaktionszeit von 2$\frac{1}{2}$ Stunden beibehalten werden kann.

Das zum Animpfen verwendete kristalline 1,8-Naphthalimid-Natrium wird aus 1,8-Naphthalimid und Natronlauge hergestellt.

Die Kristallisation des Natriumsalzes des 1,8-Naphthalimids — nach vorausgegangener Impfung der wäßig-alkalischen Lösung des 1,8-Napthalimids — während des Zulaufs der Chlorbleichlauge gewährleistet eine konstante und ausreichend niedrige Konzentration des gelösten 1,8-Naphthalimid-Natriums während der Reaktion, welche für eine reproduzierbare Lenkung der Reaktion von großer Bedeutung ist.

Verhindert man eine Kristallisation des gelösten 1,8-Naphthalimid-Natriums durch Nichtanimpfen (Vermeidung der Bildung von Kristallisationskeimen), so läuft die Reaktion, die sonst in heterogener Phase (Vorliegen des 1,8-Naphthalimid-Natriums als Bodenkörper) abläuft, in Lösung ab. Die Folge ist eine wesentliche Verringerung der Ausbeute.

Beim erfindungsgemäßen Verfahren kann Menge und Konzentration an Hypochlorit erheblich verringert werden, die Konzentration um mindestens 25%. Diese Konzentrations- und Mengen-Erniedrigung des Hypochlorits wird durch die Verwendung von Lithiumhydroxid anstelle von Natriumhydroxid beim Lösen des 1,8-Napthalimids (Herstellung der "Imidlauge") ermöglicht.

Es muß nun als überraschend erachtet werden, daß die erhebliche Herabsetzung der Hypochlorit-Konzentration und Menge entgegen den Ausführungen in der zitierten japanischen Patentschrift nicht zu einer Verlängerung der Reaktionszeit und einer damit verbundenen Qualitätsverschleichterung, sondern zu einer deutlichen Verbesserung der Qualität des Naphthostyrils bei gleichbleibender optimaler Reaktionszeit führt.

Verfährt man, wie vorstehend erfindungsgemäß beschrieben, jedoch mit der Abänderung, das 1,8-Napthalimid statt in einer Wäßriger Lösung von Lithiumhydroxid und Kaliumhydroxid in einer wäßriger Lösung von Natriumhydroxid und Kaliumhydroxid zu lösen (Herstellung der "Imidlauge"), so bleiben überraschenderweise die weiter oben beschriebenen, gegenüber dem Verfahren der zitierten japanischen Patentanmeldung gegebenen Vorteile erhalten. Die optimale Reaktionszeit verlängert sich hierbei allerdings von 2$\frac{1}{2}$ auf 4$\frac{1}{2}$ Stunden.

Nachstehend werden noch Einzelheiten und bevorzugte Ausführungsformen des erfindungsgemäßen Verfahrens angegeben;

Zweckmäßigerweise stellt man eine etwa 4-gewichtsprozentige wäßrige Lösung von Lithiumhydroxid und Kaliumhydroxid bzw. Natriumhydroxid und Kaliumhydroxid her, wobei das Molverhältnis von LiOH:KOH zweckmäßigerweise 1:1,5 beträgt, bzw. von NaOH:KOH zweckmäßigerweise so ist, daß von insgesamt 3 Mol Alkalimetallhydroxid maximal 2 Mol KOH sind. Ein zweckmäßiges Molverhältnis von NaOH:KOH ist beispielsweise 1,2:1,8.

Gemäß dem Reaktionsschema

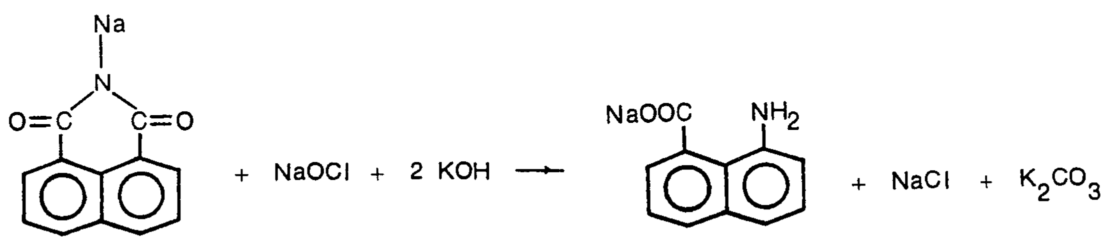


sind für den Reaktionsablauf (Hofmann'scher Abbau) 3 Mol Alkalimetallhydroxid, einschließlich der zur Alkalimetallsalzbildung des 1,8-Napthalimids benötigten Alkalimetallhydroxidmenge, erforderlich, davon 2 Mol für die Neturalisation der schließlich gebildeten Kohlensäure.

In der hergestellten wäßrig-alkalischen Lösung löst man das 1,8-Naphthalimid als Feucht- oder Trockenprodukt mit einem Midestreingehalt von 90% bei etwa 80°C. Nach vollständiger Auflösung kühlt man die Lösung auf 14°C ab, wobei keine Kristallisation eintritt. Zur Lösung gibt man dann Entschäumungs- und Dispersionsmittel. Die so erhaltene Lösung stellt die "Imidlauge" dar.

Darauf stellt man einen Kristallbrei aus Naphthalimid-Natrium her, indem man zweckmäßigerweise 2 g 1,8-Naphthalimid in 50 ml 80°C heißem Wasser mit 2 g Natriumhydroxid 100% löst und bei 20°C zu einem kristallinen Brei anreibt. Diesen Brei gibt man zu der "Imidlauge". Von den zugesetzten Impfkristallen abgesehen soll noch keine kristallisation eintreten. Sofort anschließend läßt man innerhalb von 2 Minuten unter Rühren 2,3 Mol einer 13—13,5 %igen Chlorbleichlauge zulaufen (Gehalt an Wasser 940 g). Insgesamt enthält die Mischung also 6400 g Wasser auf 200 g Napthalimid (32:1). Die Kristallisation soll bereits während des Zulaufs der Chlorbleichlauge oder unmittelbar danach einsetzen.

Nach Zulauf der Chlorbleichlauge soll die Temperatur in 5 Minuten auf 18°C ansteigen. Anschließend

wird die Reaktionsmischung 2½ Stunden bei 18°C nachgerührt. Nach ca. 1½—2 Stunden ist der Bodenkörper verschwunden.

Schon geringe Änderungen der genannten Parameter können wesentliche Einflüsse auf Ausbeute und Qualität haben.

Der Wassereinsatz sollte in den Grenzen 5—6 l, bezogen auf 1 Mol 1,8-Naphthalimid, liegen, wobei die obere Grenze unkritisch ist.

Die Alkalimetallhydroxidmenge sollte zwischen 5—6 Mol, bezogen auf 1 Mol 1,8-Naphthalimid, liegen; zu niedrige und zu hohe Mengen sind schädlich. Statt des Lithiumhydroxids kann bei der Verfahrensvariante Natriumhydroxid verwendet werden. Die Reaktionsdauer beträgt in diesem Fall 4½ Stunden (der kristalline Bodenkörper verschwindet nach ca. 4 Stunden).

Die Temperatur der Nachrührphase soll zwischen 16°C und 20°C liegen. Unterhalb von 16°C läuft die Reaktion nur sehr langsam ab, oberhalb 20°C findet in zunehmendem Maße Verharzung statt.

Der Zusatz eines Entschäumers ist empfehlenswert, um eine eventuelle Anreicherung von explosivem Stickstofftrichlorid zu vermeiden und um die Gefäßkapazität besser nutzen zu können.

Das Diespersionsmittel soll für ein gleichmäßiges Kristallwaschstum sorgen.

Die Chlorbleichlauge muß kurz vor ihrer Anwendung auf ihren Titer geprüft werden. Sie sollte bie 5°C gelagert werden und bei 10°C eingesetzt werden.

Die Einstellung der Starttemperatur von 14°C in der "Imidlauge" ist wichtig. Sie ist auf die Wärmekapazität des Reaktionsgemischs abgestimmt. Es soll dadurch gewährleistet sein, daß das Reaktionsgemisch nach dem Zulauf der Chlorbleichlauge möglichst bald (nach ca. 5 Min.) in die für die Reaktion optimale Temperaturstufe von 18°C gelangt. Die Nachrührzeit (bei Verwendung von Lithiumhydroxid) von 2½ Stunden ist möglichst genau einzuhalten. Eine Nachrührzeit von mindestens 2 bis höchstens 3 Stunden bei Verwendung von Lithiumhydroxid stellt die äußersten Grenzen der Zeitdauer der Nachbehandlung dar. Schon Zeitintervalle von 30 Minuten können merkliche Effekte bewirken;

Nach 2 Stunden ist noch Naphthalimid deutlich nachweisbar, nach 3 Stunden beginnen die Verharzungsreaktionen (erkennbar an zunehmender Verfärbung der Reaktionslösung von Hellorange nach Dunkel-rotbraun). Die optimalen Nachrührzeiten sind auch von der Qualität des eingesetzten 1,8-Naphthalimids abhängig. 90 %iges 1,8-Napthalimid verbraucht wegen der Abreaktion von gelösten Nebenkomponenten rascher Hypochlorit als 100 %iges 1,8-Naphthalimid, wodurch die Reaktionsgeschwindigkeit der Zielreaktion verlangsamt wird (um ca. 30 Minuten). Im Falle des Ersatzes von LiOH durch NaOH liegt die optimale Nachrührzeit bei 4½ Stunden.

Nach Ablauf der optimalen Nachrührzeit muß das überschüssige, als $Cl^+$ vorliegende Chlor durch Reduktion mit Sulfit zu Chlorid $Cl^-$ vernichtet werden. Dabei verbraucht 1 Mol $Cl^+$ 1 Mol $SO_3^{2-}$. Der Endpunkt der Zerstörung des überschüssigen aktiven Chlors wird durch Tüpfeln auf kaliumjodidstärkepapier erkannt. Es zeigt sich, daß man bei Anwendung von ca. 100 %igem 1,8-Napthalimid noch 0,72 Mol Sulfit benötigt, bei Einsatz von ca. 90 %igem 1,8-Naphthalimid jedoch nur 0,36 Mol Sulfit. Die dieser Differenz der benötigten Sulfitmengen entsprechende unterschiedliche Hypochloritmenge (bzw. -Konzentration) von ca. 20% äußert sich in einer Differenz von ca. 30 Minuten Nachrührzeit. Nach der Zerstörung des aktiven Chlors verfährt man im wesentlichen gemäß der Verfahrensweise der zitierten japanischen Patentanmeldung.

Anschließend heizt man die Reaktionslösung auf 90°C und stellt mit ca. 5,9 Mol 30 %iger Salzsäure auf pH 2,0, wobei Ausfällung ab pH 4,5 gemäß der Reaktionsgleichung

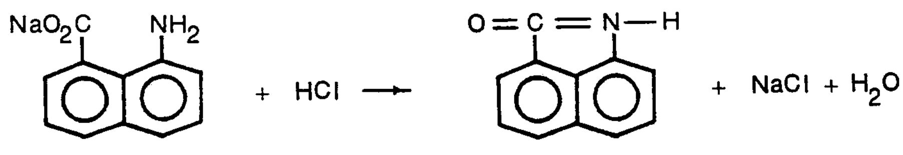

einsetzt.

Bei pH> 4,5 einsetzende Fällungen deuten auf viel Naphthalimid hin.

Man rührt die schwach saure Lösung 1 Stunden bei 90°C nach und versetzt mit einer 15 %igen Sodalösung bis zur Einstellung eines pH-Werts von 8,5 (Verbrauch ca. 0,9—1,2 Mol Soda), wodurch derjenige Anteil der Harze gelöst wird, der vorher nicht als Stippen vorlag. Produkte, die durch zu hohen Einsatz von Hypochlorit Stippen enthalten, werden durch diesen sog. "Sodaauszug" nicht von den Stippen befreit.

Man erhält 0,73 Mol (entsprechend einer Ausbeute von 73% der Theorie) Naphthostyril mit einem Reingehalt von 92% (HPLC), einem Gehalt von maximal 2% 1,8-Naphthalimid und maximal 2% Naphthalsäureanhydrid. Der Schmelzpunkt liegt bei 173°C—176°C.

Napthostyril (1,8-Napthlactam (CAS (Chemical Abstracts) Nr. [130-00-7]) ist ein wichtiges Vorprodukt zur Herstellung von 1,1'-Dinaphthyl-8,8'-dicarbonsäure (CAS Nr. [29878-91-9] und ein wichtiges Zwischenprodukt zur Herstellung von Säure- und Dispersionsfarbstoffen sowie von kationischen Farbstoffen, inbesondere Polyacrylnitril- und Polyesterfarbstoffen. Die Verwendung des Naphthostyrils wird beispielsweise beschrieben in den deutschen Offenlegungsschriften 22 37 372, 23 09 612, 23 41 657, 20 36 504 und 19 31 789 sowie in DE—AS 19 17 456, DE—PS 14 44 660 und DE—PS 12 25 326.

Das erfindungsgemäße Verfahren wird durch die nachstehenden Beispiele erläutert, ohne darauf beschränkt zu werden.

Beispiel 1

In einem 6 1-Rührkolben wird eine Lösung von 50 g Lithiumhyroxid-1-hydrat 55% (1,13 Mol LiOH) und 107 g Kaliumhydroxid 89% (1,70 Mol) in Schuppenform in 3000 ml Wasser angesetzt. In dieser Lösung werden bei 80°C 108 g Naphthalimid als wasserfeuchtes Proudkt (Reingehalt des Trockenprodukts mindestens 98% = 0, 547 Mol) völlig gelöst. Die Lösung wird auf 14°C (abgekühlt und mit 170 mg eines Entschäumers (fluorierte organische Verbindung) und 170 mg eines Dispersionsmittel (spezifischer organischer Polyelektrolyt) versetzt. Die klare Lösung der erhaltenen "Imidlauge" wird bei 14°C mit einem Kristallbrei von Naphthalimid-Natrium (aus 1,08 g 1,8-Naphthalimid +30 ml Wasser + 2,50 ml NaOH 33 %ig) versetzt. Gesamteinsatz an 1,8-Napthalimid 109,1 g (0,553 Mol).

Zu der so geimpften "Imidlauge" läßt man 680 g Chlorbleichlauge 13% (1,245 Mol aktives Chlor, entsprechend 2,26 Mol aktivem Chlor/Mol Imid), die auf 10°C eingestellt ist, in 2 Minuten unter intensivem Rühren zulaufen.

Während bzw. bis spätestens zum Ende des Zulaufs der Chlorbleichlauge setzt kristalline Fällung ein und die Temperatur steigt auf 16 bis 18°C. Man rührt bei 18°C nach. Nach ca. 85 Minuten ist der kristalline Bodenkörper verschwunden. Man rührt insgesamt 2½ Stunden (gerechnet ab Beginn des Zulaufs der Chlorbleichlauge) nach. Dann zerstört man den Überschuß an aktivem Chlor ($Cl^+$) durch Zulauf von 102 g 40 %iger wäßriger Natriumhydrogensulfit-Lösung (0,39 Mol) bei 18°C. Zusätzlich werden noch 14 g als Überschuß hinzugegeben. Man heizt die Lösung auf 90°C und rührt ½ Stunde bei 90°C nach (pH 10,6). Dann läßt man innerhalb von 15 Minuten 381 g 31 %ige Salzsäure (3,24 Mol HCl) bei 90°C bis pH 2,0 zulaufen. Bis pH 4,5 muß die Lösung klar sein. Ab pH 4,5 tritt Fällung ein. Bei pH 2,0 liegt eine helle, olivgrüne Suspension vor, die keine Stippen enthalten darf. Man rührt 1 Stunde bei 90°C nach und versetzt dann mit 463 g 15 %iger Sodalösung (0,65 Mol) bei 80°C bis pH 8,5 und rührt ½ Stunde bei 80°C nach.

Man erhält eine gelbe Suspension, die keine Stippen enthält. Dannb kühlt man auf 25°C ab und filtriert auf der Nutsche mit Vakuum. Anschließend wäscht man mit 400 ml Wasser bis zum farblosen Ablauf. Man erhält 68,5 g Naphthostyril als Feuchtprodukt (entsprechend 73% der Theorie). Der Reingehalt des Trockenprodukts (74 g) beträgt 92 bis 95%. Das Pulver hat ein hellgelbes Aussehen.

Der Schmelzpunkt liegt bei 173°C bis 176°C. Als Verunreinigung sind zu nennen: 0,8% 1,8-Naphthalimid und 1,8% 1,8-Naphthalsäureanhydrid. Außerdem sind noch ca. 2—4% Chlornaphthostyril enthalten. Aus dem Abwasser erhält man mit Salzsäure bei pH 2,0 eine Fällung von 16 g eines Feststoffs der sich als stark verunreinigtes Naphthalsäureanhyrid erweist (Gehalt ca. 70%). Damit fehlen in der Stoffbilanz noch 3% des Einsatzes, die im Abwasser verbleiben, dort jedoch biologisch abgebaut werden können.

Beispiel 2

Man verfährt, wie in Beispiel 1 beschrieben, jedoch mit der Abänderung, daß man statt Lithiumhydroxid Natriumhydroxid einsetzt (137 g 33 %ige Natronlauge (1,17 Mol)). Die Fällung des 1,8-Naphthalimid-Natriums setzt hier bereits nach den Animpfen in der "Imidlauge" ein. Nach beendetem Zulauf der Chlorbleichlauge rührt man 4½ Stunden bei 18°C nach. Der kristalline Bodenkörper verschwindet nach ca. 3½—4 Stunden. Zur Zerstörung des Überschusses an aktivem Chlor werden 89 g 40 %ige Natriumhydrogensulfitlösung (0,34 Mol) benötigt. Zur Fällung des Naphthostyrils werden 393 g 31 %ige Salzsäure benötigt. Für den Sodaauszug sind 463 g 15 %ige Sodalösung erforderlich. Ausbeute und Qualität des erhaltenen Naphthostyrils sind mit denen von Beispiel 1 identisch.

Beispiel 3

Man verfährt, wie in Beispiel 1 beschrieben, jedoch unter Verwendung eines 1,8-Naphthalimids, das aus Napthalsäure-1,8-anhydrid und gasförmigem Ammoniak auf trockenem Wege hergestellt wurde und daher stark verunreinigt ist. Der Reingehalt beträgt nur 90%.

Nach Zulauf der Chlorbleichlauge rührt man 2½ Stunden bei 18°C nach. Der kristalline Bodenkörper verschwindet nach ca. 100 Minuten. Zur Zerstörung des Überschusses an aktivem Chlor werden 68 g 40 %ige Natriumhydrogensulfit-Lösung (0,26 Mol), zur Fällung des Naphthostyrils 381 g 31 %ige Salzsäure und für den Sodaauszug 347 g 15 %ige Sodalösung benötigt.

Ausbeute und Qualität des erhaltenden Naphthostryils stimmen mit denen von Beispiel 1 überein.

Beispiel 4

Man verfährt, wie in Beispiel 1 beschreiben, jedoch mit dem Unterschied, daß nach Zulauf der Chlorbleichlauge nur 2 Stunden bei 18°C nachgerührt wird.

Zur Zerstörung des Überschusses an aktivem Chlor werden 116 g 40 %ige Natriumhydrogensulfit-Lösung (0,45 Mol) benötigt. Man erhält, wie in Beispiel 1, 74 g Napthostyril nach dem Trocknen bei 110°C. Obwohl der Reingehalt (96,0%) höher ist und damit auch die Ausbeute (76% der Theorie), enthält das Produkt noch deutlich mehr Naphthalimid (2,2%) als das gemäß Beispiel 1 erhaltene Produkt (0,8%). Der Gehalt an Anhydrid beträgt 1,8%.

Beispiel 5

Man verfährt, wie in Beispiel 1 beschrieben, jedoch mit dam Unterschied, daß für die "Imidlauge" nur 1,5 l Wasser vorgelegt werden. Selbst bei 80°C liegt das Imid als Suspension vor. Trotzdem wird auch hier, wie erfindungsgemäß üblich, bei 14°C angeimpft und mit Chlorbleichlauge versetzt. Nach 5 Stunden bei 18°C liegt eine dem äußeren Erscheinungsbild nach noch unveränderte Suspension vor. Zur Zerstörung des aktiven Chlors werden 259 g 40 %ige Natriumhydrogensulfit-Lösung (1,0 Mol) benötigt. In Abänderung der bei Beispiel 1 angegebenen Aufarbeitung wird der Bodenkörper direkt anschließend abfiltriert. Es werden 57 g verunreinigtes Napthalimid (Gehalt ca. 90%) zurückerhalten.

Beispiel 6

Man varfährt, wie in Beispiel 1 beschrieben, jedoch mit folgenden Abänderungen;

(1) Man verwendet eine gut gereinigte, von Impfkeimen freie Apparatur.

(2) Man setzt eine Naphthalimid derselben Qualität wie in Beispiel 3 ein.

(3) Es erfolgt kein Animpfen.

(4) Man rührt nur 2 Stunden bei 18°C nach.

Nach beendetem Zulauf der Chlorbleichlauge setzt keine Fällung ein. Die stärkere Wärmetönung zu Beginn der Reaktion deutet auf eine höhere Reaktionsgeschwindigkeit hin. Zur Zerstörung des Überschusses an aktivem Chlor werden 61 g 40 %ige Natriumhydrogensulfit-Lösung 40 %ig (0,24 Mol), zur Fällung des Naphthosytrils 381 g 31 %ige Salzsäure und für den Sodaauszug 347 g 15 %ige Sodalösung benötigt. Die Suspension enthält keine Stippen.

Die Ausbeute an Naphthostyril beträgt nur 62,4 g (100 %ig). Trockenprodukt 68 g Reingehalt = 91,8% (HPLC).

Die Verunreinigungen sind: 1,4% Naphthalimid, 1,9% Naphthalsäureanhydrid (HPLC). Fp 172—174°C. Der Ausbeuteverlust kommt durch einen erhöhten Anteil an sodalöslichen Harzprodukten zustande.

Beispiel 7

(Verfleichsbeispiel gemäß der JA—OS Sho-55-35-051, wobei anspruchsfreie Anteile an Beispiel 1 der vorliegenden Erfindung angepaßt wurden)

In einem 6 1-Rührkolben wirde eine Lösung von 111 g Natronlauge 33 %ig (0,91 Mol) und 58 g Kaliumhydroxid 89 %ig (0,91 Mol) in Schuppenform in 2160 ml Wasser angesetzt. In dieser Lösung werden 108 g 1,8-Napthalimid als wasserfeuchtes Produkt (Reingehalt des Trockenprodukts mindestens 98% (0,547 Mol) bis 70°C völlig gelöst. Die Lösung wird auf 14°C abgekühlt und bei dieser Temperatur bis zur Bildung von Kristallen gerührt (etwa ½ Stunde). Ein Animpfen unterbleibt.

Zu der so hergestellten "Imidlauge" läßt man 897 g Chlorbleichlauge 13 %ig (1,642 Mol aktives Chlor, entsprechend 3,0 Mol aktives Chlor pro Mol Imid), die auf 10°C eingestellt ist, innerhalb von 2 Minuten unter intensivem Rühren zulaufen. Gleichzeitig läuft eine 20°C kalte Lösung von 100 g 33 %iger Natronlauge (0,82 Mol) in 790 ml Wasser hinzu. Gegen Ende des Zulaufs setzt schlagartig eine kristalline weiße Fällung ein. Die Suspension schäumt stark. Man rührt bei 18—19°C bis zur Lösung nach, wozu 2¾ Stunden benötigt werden. Anschließend zerstört man den Überschuß an aktivem Chlor durch Zulauf von 150 g 40 %iger Natriumhydrogensulfit-Lösung (0,58 Mol) bei 18°C. Zusätzlich weden noch 14 g als Überschuß hinzugegeben. Man heizt die Lösung auf 90°C und rührt ½ Stunde bei 90°C nach (pH 10,7). Dann läßt man innerhalb von 15 Minuten 312 g 31 %ige Salzsäure (2,65 Mol HCl) bei 90°C bis pH 2,0 zulaufen. Die Lösung ist bis pH ca. 5 klar. Ab pH 4,5 tritt Fällung eine. Bei pH 2,0 liegt eine gelbe, mit dunklen Stippen durchsetzte Suspension vor. Man rührt 1 Stunde bei 90°C nach und versetzt dann mit 463 g 15 %iger Sodalösung (0,65 Mol) bis pH 8,5 bei 80°C und rührt ½ Stunde bei 80°C nach. Man erhält ein dunkelbraune Suspension. Man kühlt auf 25°C ab und filtriert auf der Nutsche mit Vakuum. Man wäscht mit 400 ml Wasser bis zum farblosen Ablauf. Man erhält 54,0 g Naphthostyril als Feuchtprodukt (58% der Theorie). Das Produkt hat ein dunkles Aussehen. Der Reingehalt des Trockenprodukts (= 71,3 g) beträgt 76%. Der Schmelzpunkt liegt bei 160°C bis 162°C.

Als Verunreinigung ist enthalten 1,8-Naphthalimid (1%) und Naphthalsäureanhydrid (14,8%).

Im HPLC wurde noch eine unbekannte Verbindung zu ca. 4% gefunden.

**Patentansprüche**

1. Verfahren zur Herstellung von Naphthostyril durch Lösen von Naphthalimid in einer wäßrigen Alkalimetallhydroxidlösung unter Erwärmen oberhalb 40°C, Abkühlen dieser Lösung auf eine Temperatur unterhalb 20°C bis zur mindestens teilweisen Kristallisation des Naphthalimids und anschließende Umsetzung mit Natriumhypochlorit bei 10—30°C in Gegenwart von Alaklimetallhydroxid, reduktive Entfernung von überschüssigem Chlor, Eninstellung eines sauren pH-Werts durch Zugabe höher konzentrierter Salzsäure, Einstellen einer Temperatur von 90—100°C bis zur Abscheidung des Naphthostyrils in kristalliner Form, dadurch gekennzeichnet, daß man 1,8-Naphthalimid in einer wäßrigen Lösung von Lithiumhydroxid und Kaliumhydroxid unter Erwärmen auf 40°C bis 100°C löst, die erhaltene Lösung auf 14°C abkühlt und bei dieser Temperatur mit maximal 1 Molprozent 1,8-Naphthalimid-Natrium, bezogen auf 1 Mol eingesetztes 1,8-Naphthalimid, impft, anschließend Chlorbleichlauge bei 10°C bis 20°C zugibt, wobei sich das Gewichtsmengenverhältnis von eingesetztem 1,8-Naphthalimid zu insgesamt

vorhandenem Wasser zwischen etwa 1:25,4 und etwa 1:32 bewegen soll, dann 2 bis 3 Stunden bei 16 bis 20°C nachrührt, nach erfolgter Umsetzung überschüssiges aktives Chlor reduktiv beseitigt, die angefallene wäßrigalkalische Lösung des Alkalimetallsalzes der gebildten 1 Amino-naphthalin-8-carbonsäure nach Hochheizen auf 90°C mit etwa 5,9 Mol etwa 30%iger Salzsäure, bezogen auf 1 Mol eingesetztes 1,8-Naphthalimid, auf den pH-Wert 2,5 bis 2,0 stellt und das hierbei ausfallende Natphthostyril isoliert.

2. Verfahren nach Anspruch 1, dadurch gekennzeichnet, daß man das 1,8-Naphthalimid in einer etwa 4 %igen wäßrigen Lösung von Lithiumhydroxid und Kaliumhydroxid löst, wobei das Molverhältnis LiOH:KOH = 1:1,5 beträgt.

3. Verfahren zur Herstellung von Naphthostyril, dadurch gekennzeichnet, daß man 1,8-Naphthalimid in einer wäßrigen Lösung von Natriumhydroxid und Kaliumhydroxid unter Erwärmen auf 40°C bis 100°C löst, die erhaltene Lösung auf 14°C abkühlt und bei dieser Temperatur mit maximal 1 Molprozent 1,8-Naphthalimid-Natrium, bezogen auf 1 Mol eingesetztes 1,8-Naphthalimid, impft, anschliebend Chlorbleichlauge bei 10°C bis 20°C zugibt, wobei sich das Gewichtsverhältnis von eingesetztem 1,8-Naphthalimid zu insgesamt vorhandenem Wasser zwischen 1:25,4 und etwa 1:32 bewegen soll, dann etwa 4½ Stunden bei 16 bis 20°C nachrührt, nach erfolgter Umsetzung überschüssiges aktives Chlor reduktiv beseitigt, die angefallene wäßrig-alkalische Lösung des Alkalimetallsalzes der gebildeten 1-Amino-naphthalin-8-carbonsäure nach Hochheizen auf 90°C mit etwa 5,9 Mol etwa 30 %iger Salzsäure bezogen auf 1 Mol eingesetztes 1,8-Naphthalimid auf den pH-Wert 2,5 bis 2,0 stellt und das hierbei ausfallende Naphthostryil isoliert.

4. Verfahren nach Anspruch 3, dadurch gekennzeichnet, daß man das 1,8-Naphthalimid in einer etwa 4 %igen wäßrigen Lösung von Natriumhydroxid und Kaliumhydroxid löst, wobei das Molverhältnis NaOH:KOH = 1,2:1,8 beträgt.

**Revendications**

1. Procédé pour préparer du naphtostyryle par dissolution de naphtalimid dans une solution aqueuse d'hydroxyde(s) de métal alcalin ou de métaux alcalins, avec chauffage au-delà de 40°C, refroidissement de cette solution à une température inférieure à 20°C jusqu'à cristallisation au moins partielle du naphtalimide puis réaction avec l'hypochlorite de sodium à 10—30°C en présence d'hydroxyde de métal alcalin, élimination par réduction de l'excès de chlore, adjustement d'un pH acide par addition d'acide chlorhydrique fortement concentré, adjustement d'une température de 90 à 100°C jusqu'à séparation du naphtostyryle sous forme cristalline, procédé caractérisé en ce qu'on dissout le naphtalimide-1,8 dans une solution aqueuse d'hydroxyde de lithium et d'hydroxyde de potassium en chauffant jusqu'à 40°C à 100°C, on refroidit à 14°C la solution ainsi obtenue et on l'ensemence à cette température avec 1 mole % au maximum de naphtalimide-1,8 sodique, pour 1 mole du naphtalimide-1,8 mis en oeuvre, puis l'on adjoute de la lessive chlorée de blanchiment, entre 10°C et 20°C, le rapport pondéral entre le naphtalimide-1,8 mis en oeuvre et l'eau totale présente devant se situer entre environ 1:25,4 et environ 1:32, puis l'on continue à agiter durant deux à trois heures entre 16 et 20°C; après achèvement de la réaction, on élimine par réduction l'excès de chlore actif, on ajuste la solution alcaline aqueuse obtenue du sel de métal alcalin de l'acide amino-1 naphtalène-carboxylique-8 formé, après chauffage à 90°C, à un pH de 2,5 à 2,0 à l'aide d'environ 5,9 moles d'acide chlorhydrique à 30% enivron, pour 1 mole du naphthalimide-1,8 mis en oeuvre, et l'on isole le naphtostyryle ainsi précipité.

2. Procédé selon la revendication 1, caractérisé en ce qu'on dissout le naphtalimide-1,8 dans une solution aqueuse à 4% environ d'hydroxde de lithium et d'hydroxyde de potassium, le rapport molaire LiOH:KOH étant de 1:1,5.

3. Procédé pour préparer du naphtostyryle, caractérisé en ce qu'on dissout le naphtalimide-1,8 dans une solution aqueuse d'hydroxyde de sodium et d'hydroxyde de potassium, en chauffant jusqu'à 40°C à 100°C, on refroidit à 14°C la solution ainsi obtenue et, à cette température, on ensemence avec au maximum 1 mole % de naphtalimide-1,8 sodique, pour 1 mole de naphtalimide-1,8 mis en oeuvre, puis l'on ajoute de la lessive chlorée de blanchiment, entre 10°C et 20°C, le rapport pondéral entrè le naphtalimide-1,8, mis en oeuvre et la quantité d'eau totale présente devant se situer entre 1:25,4 et environ 1:32, puis l'on continue à agiter durant quatre heures et demie environ entre 16° et 20°C et, une fois la réaction achevée, on élimine par réduction l'excès de chlore actif, on ajuste, après chauffage à 90°C, la solution alcaline aqueuse obtenue du sel de métal alcalin de l'acide amino-1 naphtalène-carboxylique-8 formé, à un pH de 2,5 à 2,0 à l'aide d'environ 5,9 moles d'acide chlorhydrique à 30% environ, pour 1 mole du naphtalimide-1,8 mis en oeuvre, et l'on isole le naphtostyryle ainsi précipité.

4. Procédé selon la revendication 3, caractérisé en ce qu'on dissout le naphtalimide-1,8 dans une solution aqueuse à 4% environ d'hydroxyde de sodium et d'hyroxyde de potassium, le rapport molaire NaOH:KOH étant de 1,2:1,8.

**Claims**

1. A process for the preparation of naphthostyril by dissolving naphthalimide in an aqueous alkali metal hydroxide solution with warming above 40°C, cooling this solution to a temperature below 20°C until at least partial crystallization of the naphthalimide takes place and subsequent reaction with sodium hypochlorite at 10—30°C in the presence of alkali metal hydroxide, reductive removal of excess chlorine,

adjusting the pH to an acidic value by adding hydrochloric acid of fairly high concentration, adjusting the temperature to 90—100°C until the naphthostyril precipitates in crystalline form, which process comprises dissolving 1,8-naphthalimide in an aqueous solution of lithium hydroxide and potassium hydroxide with heating to 40°C to 100°C, cooling the solution obtained to 14°C and seeding the solution at this temperature with no more than 1 mol percent of sodium 1,8-naphthalimide, relative to 1 mole of 1,8-naphthalimide used, then adding chlorine bleaching liquor at 10°C to 20°C, in which the weight ratio of 1,8-naphthalimide used to the total amount of water present should vary within the range between about 1:25.4 and about 1:32, then stirring at 16 to 20°C for another 2 to 3 hours, then, after the reaction is completed, reductively removing excess active chlorine, then, after heating the resulting aqueous alkaline solution of the alkali metal salt of the 1-aminonaphthaline-8-carboxylic acid formed to 90°C, adjusting the pH to 2.5 to 2.0 with about 5.9 mol of approximately 30% hydrochloric acid, relative to 1 mol of 1,8-naphthalimide used, and isolating the naphthostyril precipitated in this process.

2. The process as claimed in claim 1, wherein the 1,8-naphthalimide is dissolved in an approximately 4% aqueous solution of lithium hydroxide and potassium hydroxide, in which the molar ratio of LiOH to KOH is 1:1.5.

3. A process for the preparation of naphthostyril, which process comprises dissolving 1,8-naphthalimide in an aqueous solution of sodium hydroxide and potassium hydroxide with heating to 40°C to 100°C, cooling the solution obtained to 14°C and seeding the solution at this temperature with no more than 1 mol percent of sodium 1,8-naphthalimide, relative to 1 mol of 1,8-naphthalimide used, then adding chlorine bleaching liquor at 10°C to 20°C, in which the weight ratio of 1,8-naphthalimide used to the total amount of water present should vary within the range between about 1:25.4 and about 1:32, then stirring at 16 to 20°C for about 4½ hours, then, after the reaction is completed, reductively removing excess active chlorine, then, after heating the resulting aqueous alkaline solution of the alkali metal salt of the 1-amino-naphthaline-8-carboxylic acid formed to 90°C, adjusting the pH to 2.5 to 2.0 with about 5.9 mol of approximately 30% hydrochloric acid, relative to 1 mol of 1,8-naphthalimide used, and isolating the naphthostyril precipiated in this process.

4. The process as claimed in claim 3, wherein the 1,8-naphthalimide is dissolved in an approximately 4% aqueous solution of sodium hydroxide and potassium hydroxide, in which the molar ratio of NaOH to KOH is 1.2:1.8.